# EUROPÄISCHE PATENTANMELDUNG

(11) **EP 1 236 798 A2**
(43) Veröffentlichungstag der Anmeldung: **04.09.2002**
(21) Anmeldenummer: 01112165.4
(22) Anmeldetag: 17.05.2001
(51) Int. Cl.: C12N 15/10, C07K 14/47, C12N 5/10

(54) **Genbibliothek und Verfahren zu deren Herstellung**

(30) Priorität: 28.02.2001 DE 20103510 U
(71) Anmelder: LION Bioscience AG, 69123 Heidelberg (DE)
(72) Erfinder: Höfer, Michael, Dr., 69121 Heidelberg (DE); Hofmann, Martin, Dr., 69118 Heidelberg (DE); Kaiser, Carmen, Dr., 69221 Dossenheim (DE); Kranz, Harald, Dr., 69221 Dossenheim (DE); Löbbert, Ralf, Dr., 69115 Heidelberg (DE); Schlüter, Thomas, Dr., 69221 Dossenheim (DE)
(74) Vertreter: Schüssler, Andrea, Dr.

(57) **Zusammenfassung**

Beschrieben wird ein Verfahren zur Herstellung einer Genbibliothek, vorzugsweise einer cDNA-Genbibliothek, und eine durch dieses Verfahren erhältliche Genbibliothek.

## Beschreibung

Die vorliegende Erfindung betrifft ein Verfahren zur Herstellung einer Genbibliothek, vorzugsweise einer cDNA-Genbibliothek, und eine durch dieses Verfahren erhältliche Genbibliothek.

Eine Genbibliothek stellt eine Sammlung rekombinanter DNA-Moleküle in der Form von z.B. Bakterien/Plasmid-Klonen, Phagenlysaten etc. dar. Im Idealfall repräsentieren die Insertionen des Vektors die gesamte genetische Information eines bestimmten Organismus oder z.B. Gewebes, wobei die Wahrscheinlichkeit mit welcher sich ein bestimmtes Gen in einer solchen Sammlung finden läßt, von der Größe der Insertion, der Größe des Gesamtgenoms und der Anzahl der Kopien des betreffenden Abschnitts, z.B. des Gens, im Genom abhängt. cDNA-Banken bieten den Vorteil, daß nur tatsächlich exprimierte Gene enthalten sind und diese ohne Intronanteile. Darüber hinaus kann mittels cDNA-Banken die Genexpression in bestimmten Zellen und/oder Geweben in Abhängigkeit von bestimmten Faktoren, z.B. des Differenzierungsstatus untersucht werden. Allerdings taucht hier häufig - in Abhängigkeit von den für die cDNA-Synthese von der mRNA als Matrize verwendeten Primern - das Problem auf, daß nicht das jeweilige Gesamtgen in der Genbank vorhanden ist oder nicht die gewünschten Teilbereiche der Gene.

Jedenfalls sind die bisher verwendeten Verfahren zur Herstellung von cDNA-Banken aufwändig, wenig effizient und weisen eine Reihe von weiteren nachstehend diskutierten Nachteilen auf. Hierbei ist zu erwähnen, daß das Hauptproblem bei der Verwendung von herkömmlich hergestellten cDNA-Bibliotheken für die Untersuchung der Genexpression mit Hilfe von Mikroarrays in unspezifischen Kreuzreaktionen von gespotteten cDNA-Klonen, sie dies als Plasmid oder als PCR-Amplifikat, mit der Proben-RNA während der Hybridisierung liegt sowie in einem schlechten "signal to noise"-Verhältnis. Beide Faktoren können dabei zu einer völligen Fehlinterpretation der Hybridisierungsergebnisse und damit des Gesamtexperiments führen. Im einzelnen können diese Probleme auf verschiedenen Ursachen beruhen:
a) Konservierte Domänen in den kodierenden Sequenzen einzelner Vertreter einer Genfamilie führen zu einer unspezifischen Kreuzreaktion.
b) Da zur Herstellung der PCR-Amplifikate in der Regel Amplifizierungsprimer benutzt werden müssen, die auf dem verwendeten Klonierungsvektor liegen, werden normalerweise Vektoranteile mitamplifiziert. Dies kann sowohl zu einer unspezifischen Reaktion führen, als auch zu einer deutlichen Verschlechterung des Verhältnisses von "signal to noise".
c) Die normalerweise verwendeten Amplifizierungsprimer besitzen eine vergleichsweise niedrige Schmelztemperatur (Tm), dementsprechend muß die Amplifizierungsreaktion bei einer relativ niedrigen Annealingtemperatur durchgeführt werden. Das Risiko einer Fehlbindung der Amplifizierungsprimer und damit einer unspezifischen Amplifikation ist entsprechend relativ hoch.
d) Die unterschiedlich langen Poly-A-Schwänze der verschiedenen cDNA-Moleküle können sowohl zu einem stärkeren Hintergrund als auch zu einer unspezifischen Hybridisierungsreaktion führen.
e) Werden die herkömmlich hergestellten cDNA-Bibliotheken für die Herstellung von Mikroarrays verwendet, so liegen die cDNA-Integrate in der Regel ungerichtet im Polylinker eines Vektorsystems vor. Das ausschließliche Spotten des "antisense"-Stranges durch Verwendung nur eines modifizierten PCR-Primers ist damit ausgeschlossen. Dies führt wiederum zu einer deutlichen Verschlechterung des Verhältnisses von "signal to noise".
f) Die stark unterschiedlichen Größen der einzelnen cDNAs zwischen 500 bp und >5 kb führen zu sehr inhomogenen Ausbeuten in der PCR. Für ein aussagekräftiges Hybridisierungsexperiment müssen auf dem cDNA-Mikroarray allerdings homogene, äquimolare Mengen der einzelnen Klone gespottet sein.

Somit liegt der vorliegenden Erfindung im wesentlichen das technische Problem zugrunde, ein Verfahren zur Herstellung einer Genbibliothek bereitzustellen, die die vorstehend beschriebenen Nachteile nicht aufweist.

Die Lösung dieses technischen Problems wurde durch die Bereitstellung der in den Patentansprüchen gekennzeichneten Ausführungsformen erzielt.

Es wurde überraschenderweise gefunden, daß durch das in dem nachstehenden Beispiel beschriebene Verfahren eine Genbibliothek erzeugt werden kann, die gegenüber den herkömmlichen Verfahren eine Reihe von entscheidenden Vorteilen aufweist, vor allem hinsichtlich der Generierung von cDNA-Bibliotheken, die für die Untersuchung der Genexpression mit Hilfe von Mikroarrays optimiert sind. Die Eigenschaften einer mit dem erfindungsgemäßen Verfahren hergestellten Klonsammlung erweisen sich dementsprechend sowohl bei der Herstellung von cDNA-Mikroarrays als auch bei der eigentlichen Anwendung der Mikroarrays in Hybridisierungsexperimenten als vorteilhaft.

Da für die Herstellung von cDNA-Mikroarrays die cDNA-Integrate aus den rekombinanten Bakterienklonen mit der Polymerase-Ketten-Reaktion (PCR) amplifiziert werden sollten, sind hohe, homogene Ausbeuten in der PCR wünschenswert. Die Amplifikate sollten darüber hinaus möglichst keine Verunreinigungen mit anderen Amplifikaten enthalten, die auf eine unspezifische Primer-Bindung während der PCR zurückzuführen sind. Aus diesem Grund werden in dem erfindungsgemäßen Verfahren flankierend an die cDNA-Fragmente spezifische Primer-Bindungsstellen anligiert, deren Sequenzen einen erhöhten GC-Gehalt aufweisen, der eine Erhöhung der Primerbindungstemperatur ("Annealing-Temperatur") in der PCR zuläßt. Die Entstehung von unspezifischen Hintergrundprodukten ist bei erhöhten Annealing-Temperaturen ungleich unwahrscheinlicher.

Ein weiterer Vorteil der durch das erfindungsgemäße Verfahren erhältlichen cDNA-Bibliotheken im Vergleich zu anderen cDNA-Bibliotheken, die in Mikroarray-Anwendungen eingesetzt werden, ist die erzielbare sehr homogene Größenverteilung von ca. 200-600 bp, welche nicht zuletzt auch für homogene Ausbeuten in der PCR verantwortlich sind. Darüber hinaus ermöglicht die gleichmäßige Größenverteilung der cDNAs, daß bei der Herstellung von Mikroarrays bei einer gegebenen Konzentration der Ausgangslösung annähernd äquimolare Mengen z.B. auf einen Glasträger aufgebracht werden können. Eine arbeitsaufwändige individuelle Einstellung der Molarität der Spottinglösung ist nicht notwendig.

Ein weiterer Vorteil der durch das erfindungsgemäße Verfahren erhältlichen cDNA-Fragmente, der sich sowohl auf die Herstellung der Mikroarrays auswirkt, als auch auf die Hybridisierungsergebnisse Einfluß nimmt, ist die gerichtete Klonierung der cDNAs. Im Vektor liegen die cDNAs in einheitlich definierter Orientierung vor, so daß durch spezielle PCR-Primer-Modifikation und geeigneter Kopplungschemie auf den verwendeten Glasträgern selektiv nur der "Sensestrang" gekoppelt werden kann. In Standard-Hybridisierungen mit "antisense" markierter cDNA weisen solche "Einzelstrang"-Mikroarrays eine deutliche gesteigerte Sensitivität im Bindungsverhalten auf.

Eine ganze Reihe weiterer Vorteile bezieht sich direkt auf das Verhalten der in der erfindungsgemäßen Genbibliothek enthaltenen cDNA-Fragmente in der komplexen Hybridisierung zur Ermittlung der Genexpressionsstärke. Hintergrund-Hybridisierungen, die die Sensitivität des Systems herabsetzen oder unspezifische Hybridisierungen, die falsch positive Ergebnisse vorspiegeln, sind unerwünscht und können bei Verwendung von nicht geeigneten cDNA-Fragmenten das Versuchsergebnis negativ beeinflussen. Aus diesem Grund grenzen bei dem erfindungsgemäßen Verfahren die Primerbindungsstellen zur PCR-Amplifikation der cDNAs unmittelbar an das cDNA-Integrat. Es werden also keine unspezifischen Vektoranteile amplifiziert, die für eine nicht erwünschte Hintergrund-Hybridisierung ursächlich sein könnten. Bei den bisherigen cDNA-Klon-Kollektionen werden häufig PCR-Produkte erzeugt, die größere Anteile (> 100 bp) von Vektorsequenzen enthalten und damit die Spezifität für das cDNA-Amplifikat herabsetzten.

Ein entscheidender Vorteil des erfindungsgemäßen Verfahrens ist außerdem die Erzeugung von cDNA-Fragmenten, die ursprünglich vom 3'-untranslatierten Bereich der mRNA stammen. Diese Sequenzbereiche entwickelten sich aufgrund des fehlenden Selektionsdrucks in der Phylogenese zumeist sehr divergent, so daß in einer Hybridisierungsanalyse zur Genexpressionsuntersuchung auch eng verwandte Mitglieder einer Genfamilie eindeutig differenziert werden können. Kreuzhybridisierungen zwischen evolutionär stark konservierten Sequenzbereichen treten bei konventionellen cDNA-Bibliotheken bevorzugt in kodierenden Sequenzabschnitten auf. Bedingt durch die 3'-Selektion der cDNAs in Verbindung mit der stringenten Größenselektion von 200-600 bp sind in den durch das erfindungsgemäße Verfahren erhältlichen cDNA-Bibliotheken zum größten Teil 3'-untranslatierte Bereiche von Transkripten repräsentiert, die eine erhöhte Hybridisierungs-Spezifität in Mikroarray-Anwendungen erlauben. Die in konventionellen cDNA-Bibliotheken häufig vorhandenen homopolymeren poly(A)-Schwänze, die das 3'-Ende eines Transkripts markieren, werden im Verlauf des erfindungsgemäßen Verfahrens entfernt. Längere poly(A)-Sequenzen auf dem Mikroarray setzten die Spezifität der Hybridisierung herab und verursachen damit eine Verschlechterung des "signal to noise"-Verhältnisses.

Das erfindungsgemäße Verfahren basiert im wesentlichen auf folgenden Schritten:
(a) Ausgehend von mRNA, Synthese des ersten Strangs der cDNA unter Verwendung eines Primers, der an seinem 5'-Ende einen ersten Partner eines Bindungspaars, vorzugsweise kovalent gebunden, enthält, der eine Affinität zu dem zweiten Partner des Bindunsgpaars aufweist. Geeignete Bindungspaare sind dem Fachmann bekannt und dazu zählen z.B. Biotin/Streptavidin, Biotin/Avidin, Antigen/Antikörper etc. Der Primer enthält außerdem an seinem 3'-Ende eine zu dem poly(A)-Schwanz der mRNA komplementäre poly(dT)-Sequenz ausreichender Länge sowie zwischen dem 5'-Ende, an das der erste Bindungspartner geknüpft ist und zwischen der poly(dT)-Sequenz eine Sequenz, die als Doppelstrang einer Erkennungsstelle für ein Restriktionsenzym des Typs II entspricht, z.B. BpmI, wodurch die spätere Abtrennung der poly(dT)-Sequenz oder eines Teils davon von der cDNA erlaubt wird. Vorzugsweise ist der Primer ein Gemisch von Primern, die 3' zu der poly(dT)-Sequenz die Sequenz 5'-VN-3' aufweisen, wobei V A,C,G und N A,C,G oder T ist;
(b) Zweitstrangsynthese unter üblichen Bedingungen, z.B. (1) Spaltung der RNA des DNA/RNA-Hybrids mit RNAse H, (2) Herstellung des komplementären Strangs mit DNA-Polymerase I und (3) Verknüpfung der offenen Okazaki-Fragmente mit Ligase;
(c) Fragmentierung der DNAs aus Schritt (b) über bekannte Verfahren, vorzugsweise als Zufallsfragmentierung, wobei Fragmentierung über Ultraschallbehandlung bevorzugt ist;
(d) ggf. Isolierung der Fragmente mit dem gewünschten Längenbereich gemäß bekannter Verfahren, z.B. Agarosegelelektrophorese und Isolierung gewünschter Fragmente aus dem Gel. Vorzugsweise werden Fragmente im Bereich von 100 bis 1000 bp, mehr bevorzugt im Bereich von 200 bis 600 bp isoliert. Im Anschluß daran werden die DNA-Fragmente an ihren Enden preferentiell enzymatisch geglättet, d.h. Überhänge entweder des 3'-Endes oder des 5'-Endes entfernt, z.B. unter Verwendung von Pwo-DNA-Polymerase oder Pfu-DNA-Polymerase;
(e) Bindung der Fragmente, die dem 3'-Bereich der mRNA entsprechen, über den ersten Bindungspartner an den zweiten Bindungspartner, der an einen festen Träger gekoppelt ist. Geeignete feste Träger sind dem Fachmann bekannt und dazu zählen zum Beispiel paramagnetische Perlen. Dann werden die nicht-gebundenen Fragmente (ohne ersten Bindungspartner) durch Waschen entfernt;
(f) an dem dem biotinylierten Ende der Fragmente gegenüberliegenden Ende der DNA-Fragmente wird ein Adaptermolekül über gängige Verfahren ligiert, das in seiner 5'-überstehenden Sequenz eine erste Restriktionsstelle für ein Restriktionsenzym enthält, das vorzugsweise so schneidet, daß überstehende DNA-Enden erzeugt werden (siehe dazu auch beispielhaft den Adapter in dem nachstehenden Beispiel);
(g) mit den immer noch an den festen Träger gebundenen Fragmenten wird eine PCR durchgeführt, wobei folgende Primer verwendet werden: Der 5'-Primer ist ein Primer, der im wesentlichen mit der Sequenz des überstehenden Endes des Adapters (oder einem Teil davon) identisch ist. Der 3'-Primer ist an seinem 5'-Ende mit einem ersten Bindungspartner eines Bindungspaares verknüpft, der dem von Schritt (a) entsprechen kann oder unterschiedlich sein kann und seine Sequenz entspricht im wesentlichen der des Primers für die Erststrangsythese einschließlich der Restriktionsschnittstelle für ein Restriktionsenzym des Typs II. Somit führt in der PCR der 3'-Primer zur Synthese eines Strangs, dessen Sequenz im wesentlichen dem des ersten cDNA-Strangs entspricht. An diesen lagert sich dann der 5'-Primer an, der dann zur Synthese des dazu komplementären Strang unter Einführung der Restriktionsschnittstelle des Adapters führt;
(h) nach Beendigung der PCR und vorzugsweisen Reinigung der Amplifikationsprodukte wird mit dem passenden Restriktionsenzym des Typs II ein Verdau durchgeführt, wobei die poly(dT)-Schwänze abgeschnitten werden. Vorzugsweise verbleiben noch zwei bis vier dT-Reste an den Fragmenten. Die abgeschnittenen poly(dT)-Schwänze werden dann durch Bindung ihres ersten Bindungspartners an den an einen festen Träger gekoppelten zweiten Bindungspartner von den Fragmenten ohne Bindungspartner abgetrennt;
(i) die von den poly(dT)-Schwänzen befreiten PCR-Fragmente (ohne Bindungspartner) werden vorzugsweise nochmals hinsichtlich ihrer Länge überprüft und Fragmente im gewünschten Längenbereich (siehe Schritt (d)) isoliert. Danach erfolgt die Ligation eines Adapters an das Ende der Fragmente, von dem die poly(dT)-Schwänze abgeschnitten wurden, vorzugsweise eines Adapters, der an einem Ende ein überstehendes d(TT) ausweist und somit mit dem d(AA)-Überhang der bevorzugten PCR-Fragmente aus Schritt (h) paaren kann. Das andere überstehende Ende des Adapters trägt eine Erkennungsstelle für ein zweites Restriktionsenzym, das vorzugsweise überstehende Enden erzeugt, wobei sich die Erkennungsstelle vorzugsweise von der ersten Erkennungsstelle des ersten Adaptermoleküls aus Schritt (f) unterscheidet; zur möglichen Struktur dieses Adapters wird auf das nachstehende Beispiel verwiesen; und
   im letzten Schritt werden die Fragmente aus Schritt (i) mit dem Restriktionsenzym für die erste Erkennungsstelle geschnitten (die Schnittstelle für das zweite Restriktionsenzym ist bereits "offen") und die Fragmente werden in einen entsprechend geschnitten Vektor, vorzugsweise gerichtet, ligiert. Dabei muß es sich nicht um den für die eigentliche Genbibliothek verwendeten Vektor handeln, sondern dies kann auch ein "Zwischenvektor" sein. Mittels diesem wird das Ligationsgemisch in einem geeigneten Wirt, z.B. E.coli, gemäß Standardverfahren amplifiziert; siehe z.B. Maniatis et al., 1989, Molecular Cloning: A Laboratory Manual, Cold Spring Harbor Laboratory, Cold Spring Harbor, NY. Nach der in vivo-Amplifikation wird die Vektor-DNA aus den Transformanten isoliert und in den endgültigen Wirt zur Etablierung der endgültigen Genbibliothek eingeschleust; siehe z.B. den Übersichtsartikel Current Protocols in Molecular Biology, 2. Ausgabe, 1988, Herausgeber Asubel et al., Greene Publish Assoc. & Wiley Interscience, Kapitel 13.

Somit betrifft die vorliegende Erfindung ein Verfahren zur Herstellung einer Genbibliothek, wobei das Verfahren die folgenden Schritte umfaßt:
a) Herstellen einer doppelsträngigen cDNA aus einer mRNA-Population, wobei für die Synthese des ersten cDNA-Strangs ein Primer verwendet wird, der an seinem 5'-Ende einen ersten Bindungspartner eines Bindungspaars enthält, der eine Affinität zu einem zweiten Bindungspartner des Bindungspaars aufweist, an seinem 3'-Ende eine zu dem poly(A)-Schwanz der mRNA komplementäre poly(dT)-Sequenz und zwischen dem 5'-Ende, an das der erste Bindungspartner geknüpft ist und der poly(dT)-Sequenz eine Sequenz, die als Doppelstrang eine Erkennungsstelle für ein Restriktionsenzym des Typs II umfaßt;
b) Fragmentieren der in Schritt (a) erhaltenen cDNA;
c) ggf. Isolieren der Fragmente mit der gewünschten Länge aus Schritt (b);
d) ggf. Versehen der (isolierten) Fragmente mit glatten Enden;
e) Binden der Fragmente aus dem jeweils vorangegangenen Schritt an den zweiten Bindungspartner, der an einen festen Träger gebunden ist;
f) Ligieren eines doppelsträngigen Adaptermoleküls an das dem Ende mit dem ersten Bindungspartner gegenüberliegende Ende des Fragments, wobei das Adaptermolekül eine Schnittstelle für ein zweites Restriktionsenzym enthält;
g) Durchführen einer in vitro-Amplifikation der an den Träger gebundenen Fragmente mit einem Primerpaar, wobei der erste Primer des Primerpaars im wesentlichen zu einem Strang des Adaptermoleküls komplementär ist und der zweite Primer des Primerpaars an seinem 5'-Ende einen ersten Bindungspartner umfaßt, der eine Affinität zu einem zweiten Partner eines Bindungspaars aufweist, und dessen Sequenz im wesentlichen der Sequenz des Primers aus Schritt (a) oder einer dazu komplementären Sequenz entspricht;
h) ggf. Spalten der Amplifikationsprodukte aus Schritt (e) mit einem Restriktionsenzym des Typs II, das die in Schritt (a) eingeführte Restriktionsschnittstelle erkennt;
i) ggf. Ligieren eines Adaptermoleküls an das 5'-Ende der PCR-Produkte, das nach Spaltung mit dem Restriktionsenzym des Typs II gemäß dem vorherigen Schritt erhalten wurde;
j) ggf. Spalten der Produkte aus Schritt (i) mit einem zweiten Restriktionsenzym, das die in Schritt (f) eingeführte Schnittstelle erkennt, wobei die Schritte (i) und (j) auch in umgekehrter Reihenfolge durchgeführt werden können; und
k) Ligieren der Produkte aus dem jeweils vorhergegangenen Schritt in einen Vektor.

Die zeitliche Reihenfolge einzelner oder mehrerer Schritte des erfindungsgemäßen Verfahrens muß nicht grundsätzlich in der angegebenen Reihenfolge erfolgen, sondern kann u.U. variiert werden. Desweiteren sind die angegebenen Schritte c), d) und h)-j) als optional zu verstehen. Die Herstellung des ersten und zweiten Strangs der cDNA erfolgt nach Standardverfahren unter Verwendung üblicher Enzyme und Puffer und der Fachmann kennt auch geeignete Verfahren zur Isolierung von RNA aus einer Probe, wobei vorzusgweise die mRNA, z.B. über eine Oligo(dT)-Säule, angereichert wird. Die Länge der pol(dT)-Sequenz des Primers für die Erststrangsynthese ist nicht kritisch und liegt üblicherweise im Bereich von 15 bis 30, bevorzugt zwischen 18 und 21. Bevorzugt für die Erststrangsynthese ist ein Gemisch von Primern, die 3' zu der poly(dT)-Sequenz die Sequenz 5'-VN-3' aufweisen, wobei V A, C oder G ist und N A, C, G oder T.

Der Fachmann kennt auch geeignete Bindungspartner des Bindungspaars, z.B. Antigen/Antikörper, Antikörper/Digoxigenin, Biotin/Avidin und Biotin/Streptavidin, wobei Biotin/Avidin und Biotin/Streptavidin bevorzugt sind.

Bei der Erkennungsstelle für das Restriktionsenzym des Typs II kann es sich um jede Erkennungsstelle für ein Restriktionsenzym des Typs II handeln, z.B. BpmI, AlwI, BpsI, BpvI, Bci VI, BsaI, Bse RI, BsgI, Bsm Al, EarI, FokI, HgaI, HphI, MboII, PleI, SapI, SfaNI, wobei BmpI bevorzugt ist.

Die Fragmentierung der synthetisierten cDNAs erfolgt so, daß möglichst viele Fragmente in einem gewünschten Längenbereich erhalten werden. Geeignete Fragmentierungsverfahren sind dem Fachmann bekannt, wobei eine Zufallsfragmentierung bevorzugt ist. Besonders bevorzugt ist eine Fragmentierung mittels Ultraschall, wobei der Fachmann in Testreihen, die einer präparativen Fragmentierung vorausgehen, durch Variation von Parametern wie Beschallungsdauer und/oder Intensität die Bedingungen ermitteln kann, die zu einer Fragmentierung im gewünschten Längenbereich führen. Desweiteren eignen sich erfindungsgemäß enzymatische Verdaus mit Restriktionsenzymen, die 4-er, 6-er oder 8-er Fragmente erkennen, zur Fragmentierung.

Die Isolierung von Fragmenten im gewünschten Längenbereich unter Abtrennung kürzerer bzw. längerer Fragmente kann, falls gewünscht, nach jedem Verfahren, bei dem Nukleinsäure-Fragmente gemäß ihrer Länge, vorzugsweise unter nicht-denaturierenden Bedingungen, aufgetrennt werden, erfolgen, z.B. über Gelelektrophorese, Kapillargelelektrophorese, selektive Ammoniumacetat/EtOH-Fällung, wobei Gelelektrophorese bevorzugt ist. Besonders bevorzugt ist Agarosegelelektrophorese. Während oder nach Beendigung der Gelelektrophorese können z.B. die Banden angefärbt, ausgeschnitten und mittels üblicher Verfahren aus dem Gel isoliert werden.

In einer bevorzugten Ausführungsform des erfindungsgemäßen Verfahrens weisen die cDNA-Fragmente eine Länge im Bereich von 100 bis 1000 bp, mehr bevorzugt im Bereich von 200 bis 600 bp auf.

Der Fachmann kennt auch Verfahren, die es erlauben, die isolierten cDNA-Fragmente, deren Enden nach der Fragmentierung überstehend sein können, mit glatten Enden zu versehen. Dies kann mittels üblicher enzymatischer Verfahren erfolgen, wobei allerdings nur solche Vorgehensweisen für das erfindungsgemäße Verfahren geeignet sind, bei denen das poly(dA)/(dT)-Ende der Fragmente mit dem ersten Bindungspartner nicht verändert wird. Ein geeignetes Verfahren ist z.B. die Behandlung mit Pwo-DNA-Polymerase oder Pfu-DNA-Polymerase. Je nach Fragmentierungsverfahren kann das "Glätten" auch entfallen. Wenn man beispielsweise enzymatisch fragmentiert hat, muß nicht "geglättet" werden, da der Adapter aus Schritt f) auch "strictly" ligiert werden kann.

In dem erfindungsgemäßen Verfahren ist der zweite Bindungspartner vorzugsweise an einen festes Träger, z.B. einen Objektträger, eine Mikrotiterplatte, an Perlen aus organischen (z.B. Agarose oder Polyacrylamid) oder anorganischen Verbindungen etc. gebunden, wobei paramagnetische Perlen bevorzugt sind. Der Fachmann kennt auch Verfahren zur Kopplung des zweiten Bindungspartners an den festen Träger. Der Fachmann kennt - entsprechend dem gewählten Bindungspaar - auch Bedingungen, die eine Bindung der cDNA-Fragmente über ihren ersten Bindungspartner an den zweiten Bindungspartner und das Abtrennen der nicht-gebundenen Fragmente aus dem Gemisch erlauben.

Geeignete doppelsträngige Adaptermoleküle und Verfahren zur "blunt-end"-Ligation der glatten Enden der Adaptermoleküle an die glatten Enden der cDNA-Fragmente sind dem Fachmann bekannt. Das Adaptermolekül enthält eine Erkennungsstelle für ein Restriktionsenzym, wobei es sich bei dem Restriktionsenzym vorzugsweise um ein Restriktionsenzym handelt, das überstehende Enden erzeugt und dessen Erkennungsstelle eine Länge von mindestens 6 bp, bevorzugt 8 bp, aufweist, damit ausgeschlossen bzw. die Wahrscheinlichkeit stark verringert wird, das es zu einem späteren Zeitpunkt des erfindungsgemäßen Verfahrens zu einer unerwünschten Spaltung innerhalb der cDNA-Fragmente kommt. Bevorzugte Restriktionsenzyme sind AscI, NotI, SrfI, PacI, PmeI, SwaI sowie weitere dem Fachmann bekannte Restriktionsenzyme, die eine 6er bzw. 8er Sequenz erkennen. Mit den an den festen Träger gebundenen cDNA-Fragmenten wird vorzugsweise eine in-vitro-Amplifikation durchgeführt, um ausreichendes Material für die nachfolgenden Verfahrensschritte zu erhalten. Geeignete in vitro-Amplifikationsverfahren sind dem Fachmann bekannt, wobei ein bevorzugtes Amplifikationsverfahren PCR ist. Die Sequenzen der Primer, die im wesentlichen zu einem Strang des Adaptermoleküls bzw. zu der Sequenz des Primers aus Schritt (a) oder einer dazu komplementären Sequenz komplementär sind, müssen jedenfalls so gewählt sein, daß der eine Primer zu dem Ende des Verlängerungsprodukts des anderen Primers komplementär ist und somit eine exponentielle Amplifikation erfolgen kann.

In einer besonders bevorzugten Ausführungsform des erfindungsgemäßen Verfahrens erfolgt die Spaltung mit einem Restriktionsenzym in Schritt (h) so, daß das erhaltene Fragment ein überstehendes Ende mit mindestens 2 Adeninresten aufweist und wobei das Adaptermolekül in Schritt (i) bevorzugt ein dazu komplementäres überstehendes Ende mit den entsprechenden dT-Resten aufweist. Ein Fragment mit zwei überstehenden Adeninresten wird z.B. bei Verdau mit BmpI erhalten.

Am Ende des erfindungsgemäßen Verfahrens erfolgt die Ligation, vorzugsweise gerichtete Ligation, der cDNA-Fragmente in einen geeigneten Vektor. Beispiele solcher Vektoren sind dem Fachmann bekannt. Vorzugsweise werden die vorstehend beschriebenen DNA-Fragmente in einen Expressionsvektor kloniert. Im Falle eines Expressionsvektors für E. coli sind dies z. B. pGEMEX, pUC-Derivate (z.B. pUC8), pBR322, pBlueScript, pGEX-2T, pET3b und pQE-8. Für die Expression in Hefe sind z.B. pY100 und Ycpadl zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A. Falls man keine Expression wünscht, kann man auch gegen den "frame" in die Vektoren klonieren. Die DNA-Fragmente sind im Vektor mit regulatorischen Elementen funktionell verknüpft, die deren Expression in prokaryotischen oder eukaryotischen Wirtszellen erlauben. Solche Vektoren enthalten neben den regulatorischen Elementen, beispielsweise einem Promotor, typischerweise einen Replikationsursprung und spezifische Gene, die die phänotypische Selektion einer transformierten Wirtszelle erlauben. Zu den regulatorischen Elementen für die Expression in Prokaryonten, beispielsweise E.coli, zählen der lac-, trp-Promotor oder T7-Promotor, und für die Expression in Eukaryonten der AOX1- oder GAL1-Promotor in Hefe, und der CMV-, SV40-, RVS-40-Promotor, CMV- oder SV40-Enhancer für die Expression in tierischen Zellen. Weitere Beispiele für geeignete Promotoren sind der Metallothionein I- und der Polyhedrin-Promotor. Zu geeigneten Vektoren zählen insbesondere auch auf T7 basierende Expressionsvektoren für die Expression in Bakterien (Rosenberg et al., Gene 56(1987), 125) oder pMSXND für die Expression in Säugerzellen (Lee und Nathans, J.Biol.Chem. 263 (1988),3521). Die Einschleusung der vorstehenden Vektoren in die Wirtszelle kann mittels bekannter Verfahren durchgeführt werden, z.B. mittels Kalziumphosphat-Transfektion, DEAE-Dextran-vermittelter Transfektion, über kationische Lipide vermittelte Transfektion, Elektroporation, Infektion, "Gene-Gun" etc. Diese Verfahren sind in Standardwerken der Molekularbiologie beschrieben.

Die vorliegende Erfindung betrifft auch eine Genbibliothek, die durch das erfindungsgemäße Verfahren erhältlich ist. In einer bevorzugten Ausführungsform ist die erfindungsgemäße Genbibliothek dadurch gekennzeichnet, daß mindestens eine Sequenz eines Gens oder eines Teils davon anwesend ist, das für ein Protein kodiert, das bei einem der folgenden Prozesse eine Rolle spielt: Aminosäuresynthese, zellulärer Metabolismus, Energiemetabolismus, Fettsäure- und Phospholipidmetabolismus, Purin-, Pyrimidin-, Nukleosid- und Nukleotidaufbau und -abbau, DNA-Replikation, Transkription, Translation, Proteintransport oder Proteinbindung.

In einer mehr bevorzugten Ausführungsform ist die erfindungsgemäße Genbibliothek dadurch gekennzeichnet, daß Sequenzen von mindestens 50 Genen oder Teilen davon, vorzugsweise von mindestens 500 Genen oder Teilen davon und noch mehr bevorzugt von mindestens 800 Genen oder Teilen davon anwesend sind, deren Produkte bei gleichen oder unterschiedlichen vorstehend definierten Prozessen eine Rolle spielen.

Die erfindungsgemäße Genbibliothek ist dadurch gekennzeichnet, daß mindestens 95% der Sequenzen der anwesenden Gene zwischen 200 und 600 Basenpaaren lang sind. Der Fachmann kann durch geeignete Verfahren diese Zusammensetzung der Genbibliothek erreichen, z.B. durch Isolierung von Fragmenten in den Zwischenschritten des vorstehenden Verfahrens gemäß dieser Längen, z.B. über Agarosegelelektrophorese und Elution der Fragmente im Bereich von 200 bis 600 bp aus dem Gel gemäß Standardverfahren.

In einer weiteren bevorzugten Ausführungsform ist die erfindungsgemäße Genbibliothek dadurch gekennzeichnet, daß die Gene aus Maus, Ratte, Hund, Mensch, Schwein, Hamster oder Kuh stammen. Geeignete Quellen für diese Gene sind dem Fachmann bekannt.

In einer noch mehr bevorzugten Ausführungsform ist die erfindungsgemäße Genbibliothek dadurch gekennzeichnet, daß (a) mindestens 60%, vorzugsweise mindestens 80% und noch mehr bevorzugt mindestens 90% der Sequenzen Gene oder Teile davon umfassen, die aus dem 3'-Bereich der mRNA stammen oder (b) mindestens 60% der Gene oder Teile davon, vorzugsweise mindestens 80% und noch mehr bevorzugt mindestens 90% Sequenzen umfassen, die keinen poly(A)-Schwanz enthalten.

Noch mehr bevorzugt ist eine erfindungsgemäße Genbibliothek, die dadurch gekennzeichnet ist, daß die Sequenzen der Gene oder der Teile davon in einem prokaryontischen Plasmid vorhanden sind. Bezüglich geeigneter Plasmide wird auf die vorstehenden Ausführungsformen verwiesen.

In einer noch mehr bevorzugten Ausführungsformen ist die erfindungsgemäße Genbibliothek dadurch gekennzeichnet, daß Sequenzen von mindestens 50 Genen oder Teilen davon und bevorzugt 200 Genen oder Teilen davon und am meisten bevorzugt von 500 Genen oder Teilen vorhanden sind, ausgewählt sind:
(a) aus den Sequenzen der SEQ.ID.-Liste "Replication",
(b) aus den Sequenzen der SEQ.ID.-Liste "Transcription",
(c) aus den Sequenzen der SEQ.ID.-Liste "Translation",
(d) aus den Sequenzen der SEQ.ID.-Liste "Transport- und Bindeproteine", und
(e) aus Kombinationen von mindestens zwei der Gruppen (a) bis (d).

Die Einzelsequenzen sind im Sequenzprotokoll angegeben, das die SEQ. ID No. 1 bis SEQ. ID. No. 840 umfaßt und Bestandteil der Anmeldung ist.

Die vorliegende Erfindung betrifft auch die vorstehend beschriebene Genbibliothek enthaltende Transformanten. Zu diesen Transformanten zählen Bakterien, Hefe, Insekten- und Tierzellen, vorzugsweise Säugerzellen. Bevorzugt sind die E. coli Stämme HB101, DH1, DH10B, x1776, JM101, JM109, BL21, XL1Blue und SG 13009, der Hefestamm Saccharomyces cerevisiae und die tierischen Zellen L, 3T3, FM3A, CHO, COS, Vero, HeLa sowie die Insektenzellen sf9. Verfahren zur Transformation dieser Wirtszellen, zur phänotypischen Selektion von Transformanten und zur Expression der in der Genbibliothek enthaltenen DNA-Sequenzen unter Verwendung der vorstehend beschriebenen Vektoren sind auf dem Fachgebiet bekannt.

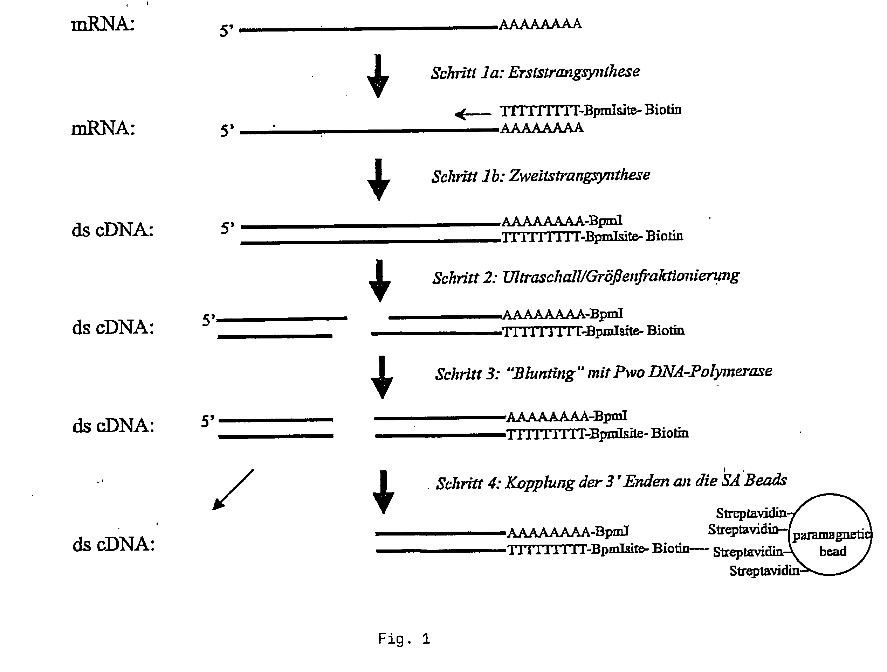

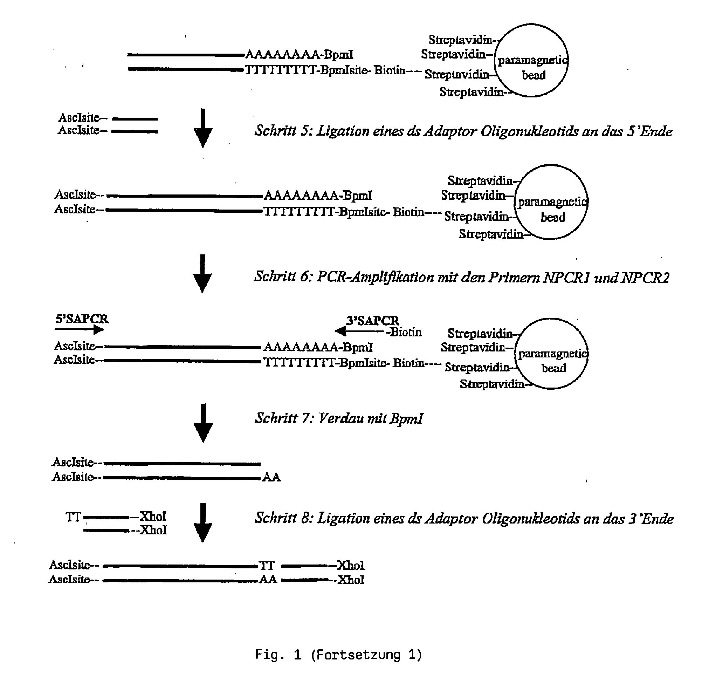

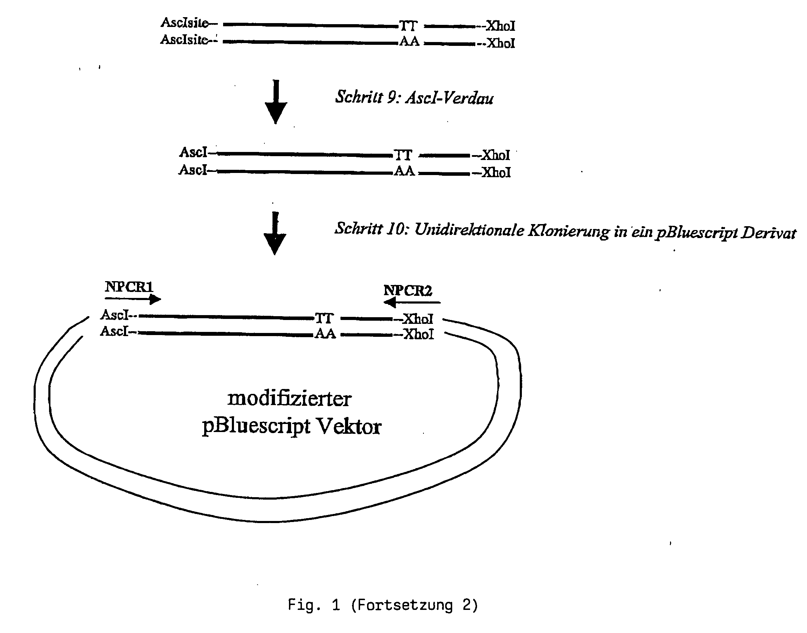

### Figur 1: Schematische Darstellung der einzelnen Schritte der Erzeugung einer cDNA-Genbibliothek wie in dem nachstehenden Beispiel beschrieben.

Das nachfolgende Beispiel veranschaulicht die Erfindung.

### Beispiel

### Herstellung einer Genbank aus mRNA von Mäuseleber

### (A) Gewinnung von mRNA

Die RNA wurde aus homogenisierter Mäuseleber unter Verwendung eines Kits für RNA-Isolierung (Trizol, Life Trchnologies, Rockville, USA) und danach eines Kits für mRNA-Isolierung (Dynabeads mRNA Purification Kit, Dynal A.S., Oslo, Norwegen) entsprechend den Empfehlungen des Herstellers extrahiert. Für die Konstruktion der Genbibliothek wurden insgesamt ca. 2 µg mRNA verwendet.

### (B) Herstellung einer doppelsträngigen cDNA

Die Erststrang-cDNA-Synthese mit dem Gibco "cDNA synthesis system" (Fa. GibcoBRL Life Technologies GmbH, Karlsruhe; Kat.Nr. 18267-013) erfolgte nach Angaben des Herstellers mit dem permutierten Primer 5'-ATG ATG CTG GAG TTT TTT TTT TTT TTT TTT VN-3', wobei V A, C oder G ist und N A,C,G,T. Der Primer trug außerdem an seinem 5'-Ende einen Biotinrest. Die unterstrichenen Nukleotide entsprechen einer BpmI-Schnittstelle (Typ II-Restriktionsenzym). Nach Verdau mit RNAse H wurde DNA-Polymerase I zur Zweitstrang-Synthese und Ligase zur Verknüpfung der offenen Okazaki-Fragmente gemäß üblicher Bedingungen verwendet. Danach wurde die erzeugte doppelsträngige DNA ethanolgefällt, getrocknet und in Puffer resuspendiert.

### Fragmentierung der cDNA durch Ultraschall

Die cDNA wurde für 1 Minute mit dem Misonex 2020 System (Fa. Misonix, Farmingdale, NY, USA) mit einem Puls von 0,9 ultraschallbehandelt. Die erfolgreiche Beschallung wurde durch Gelelektrophorese eines Aliquots überprüft. Nach erfolgreicher Kontrolle der Aliquots wurde der gesamte Ansatz mittels Agarose-Gelektrophorese aufgetrennt und Fragmente in dem Größenbereich, der der für die herzustellende Genbank gewünschten Insertlänge von 200 bis 600 bp entsprach, aus dem Agarosegel mit Hilfe des "QIAquick gel extraction kit" (Qiagen) eluiert.

### (D) Versehen der eluierten cDNA-Fragmente mit glatten Enden

Nach der Ultraschallbehandlung besitzen die isolierten DNA-Fragmente auch undefinierte 5'- oder 3'-überstehende Enden. Diese wurden mit Pwo-DNA-Polymerase (Hofmann LaRoche, Basel, Schweiz) auf glatte Ende aufgefüllt bzw. abgedaut. Dazu wurden 30 µl der DNA-Lösung mit 8 µl eines dNTP-Gemischs (200 µM pro dNTP), 2 µl destilliertem Wasser, 4,5 µl 10 x Pwo-Puffer (mit MgSO₄) sowie 0,5 µl (5 E/µl) Pwo-DNA-Polymerase versetzt und 30 Minuten bei 70°C inkubiert. Die Reaktion wurde anschließend auf Eis gestoppt.

### (E) Kopplung der 3'-Enden an Streptavidin-beschichtete paramagnetische Perlen ("SA-Beads")

Über das biotinylierte 5'-Ende der Fragmente (entsprechend dem 3'-Ende der mRNA) wurden diese von den nicht-biotinylierten Fragmenten (entsprechend internen oder 5'-Bereichen der mRNA) durch Bindung an paramagnetische Perlen (Dynabeads M-280 Streptavidin; Fa. Dynal, Oslo, Norwegen) entsprechend den Angaben des Herstellers gebunden. Durch den darauffolgenden Waschschritt wurden die übrigen Fragmente (ohne Biotinylierung) entfernt.

### (F) Ligation eines doppelsträngigen Adapter-Oligonukleotids

Im Anschluß daran wurde an das dem biotinylierten Ende gegenüberliegende Ende der noch an die Perlen gebundenen DNA-Fragmente unter Standardbedingungen ein doppelsträngiges Adapter-Oligonukleotid mit einer internen AscI-Restriktionsschnittstelle und folgender Sequenz "blunt-end"-ligiert: 5'-CTA ATA CGA CTC ACT ATA G**GG CGC GCC** AGC GTG GTC GCG GCC GAG GT-3' ;3'CAG CGC CGG CTC CA-5'. Die unterstrichene Sequenz entspricht der T7-Promotorsequenz, die fett gedruckte Sequenz GGCGCGCC der 5'-Restriktionsstelle AscI, die für die Klonierung in den Plasmidvektor verwendet wurde, und die Sequenz ACCTCGGCCGCGAC dem komplementären "Helfer"-Olignukleotid. Die Reaktion wurde über Nacht bei 16°C in einem Volumen von 20 µl durchgeführt. Der Reaktionsansatz enthielt 1 µl der gebundenen DNA, 2µl T4-DNA-Ligase (40 E/µl; Roche) und 5µl doppelsträngiges Adapter-Oligonukleotid (Endkonzentration: 2,5 µM).

### (G) Direkte PCR-Amplifikation der 3'-Enden an den "SA-Beads"

Um ausreichendes Material für die nachfolgenden Reaktionsschritte zu erhalten, wurden die immer noch an die paramagnetischen Perlen gebundenen, den 3'-Enden der Transkripte entsprechenden Fragmente über PCR amplifiziert, wobei die folgenden Primer verwendet wurden: 5'-PCR-Primer (5'SAPCR): 5'-CTA ATA CGA CTC ACT ATA GGG C-3'; 3'-Primer (3'SAPCR): Biotin-5'-ATG ATG CTG GAG TTT TTT TTT TTT TTT T-3'. Dabei liegt der 5'-PCR-Primer auf dem anligierten Adapter, der biotinylierte 3'-PCR liegt auf dem Erststrangsynthese-Primer (einschließlich der BpmI-Schnittstelle). Zuerst wurde für ein Aliquot der Reaktion die optimale Anzahl an PCR-Zyklen bestimmt, die dann später für den präparativen Ansatz verwendet wurden. Dazu wurden 10 µl des Ligationsansatzes als Matrize verwendet, 2 µl Pwo-DNA-Polymerase (Roche), je ein 1 µl der beiden Amplifizierungsprimer "5'SAPCR" (10 µM) und "3'SAPCR" (10 µM) und 10 µl dNTP-Lösung (Konzentration der einzelnen dNTPs: 1 mM). Die Reaktion wurde in einem Volumen von 50 µl in einem "DNA Thermal Cycler" (GeneAmp PCR System 9700; Perkin Elmer Applied Biosystems, Weiterstadt) durchgeführt. Nach einem einleitenden Denaturierungsschritt (5 Min., 75°C; 30 Sek., 94°C) wurden insgesamt 30 Zyklen mit folgendem Profil durchgeführt: 10 Sekunden bei 94°C, 30 Sekunden bei 60°C und 90 Sekunden bei 72°C. Anschließend erfolgte ein abschließender Elongationsschritt bei 72°C für 5 Minuten. Nach Beendigung der PCR-Reaktion wurden die PCR-Produkte mittels des "Quiaquick PCR Purification Kit" (Qiagen) aufgereinigt.

### (H) Verdau der PCR-Produkte mit dem Restriktionsenzym BpmI (Typ IIS)

Anschließend wurde ein Verdau mit dem Restriktionsenzym BmpI (NEB, Beverly, MA, USA) durchgeführt. Da während der Erstrangsynthese (siehe (A)) eine BpmI-Retsriktionsschnittstelle in direkter Nachbarschaft zu dem Poly(T)-Schwanz der cDNA eingebaut wurde, werden bei dem durchgeführten Restriktionsverdau zum einen die cDNA-Moleküle von den "SA-Beads" abgetrennt, zum anderen werden die Poly(T)-Schwänze der PCR-Produkte bis auf zwei T-Reste von den den 3'-Enden der Transkripte entsprechenden Enden der DNA-Fragmente abgeschnitten. Die "SA-Beads", an denen noch die Poly(T)-Schwänze hängen, werden von den jetzt frei in der Lösung vorliegenden, den 3'-Enden der mRNA entsprechenden cDNA-Fragmenten abgetrennt. Die Fragmente wurden wieder auf ein 1% Agarosegel aufgetragen und Fragmente mit einer Länge von 200 bis 600 bp ausgeschnitten und wie vorstehend beschrieben eluiert.

### (I) Ligation eines doppelsträngigen Adaptermoleküls an die den 3'-Enden der mRNA entpsrechenden cDNA-Moleküle

Im nächsten Schritt wurde ein 3'-TT-Adapter an das freigewordene Ende der Fragmente, das dem 3'-Ende der mRNA entspricht, anligiert. Dieser Adapter weist an seinem 3'-Ende eine "offene" XhoI-Schnittstelle auf. Der Adapter hatte folgende Sequenz: 5'-TCG AGC GGC CGC CCG GGC AGG TTT-3'; 3'-CG CCG GCG GGC CCG TCC A- -5'. Dieser Adapter enthält links eine offene XhoI-Schnittstelle und rechts ein überstehendes Ende mit TT, das mit den 3'-überstehenden AA der cDNAs kompatibel ist. Der untere Adapter ist an seinem 5'-Ende phosphoryliert. Die Reaktion wurde über Nacht bei 16°C in einem von Volumen von 100 µl durchgeführt, wobei 30 µl DNA-Lösung, 2 µl T4 DNA-Ligase (400 E/µl; Roche) und 20 µl der doppelsträngigen Adapter-DNA (210 µM) verwendet wurden. Nach dem Annealing der beiden komplementären Oligonukleotide des Adapters entstehen auf der einen Seite zwei überhängende TT-Nukelotide, die eine Ligation an die mit BpmI verdaute cDNA ermöglichen, auf der anderen Seite entsteht eine geöffnete XhoI-Schnittstelle, die später für die unidirektionale Klonierung verwendet wurde.

### (J) Verdau der cDNA-Fragmente mit AscI

Anschließend wurde durch Verdau der so erhaltenen ligierten Fragmente mit AscI (NEB) die AscI-Schnittstelle geöffnet. Die Fragmente trugen jetzt an ihrem 5'-Ende eine AscI-Schnittstelle und an ihrem 3'-Ende eine XhoI-Schnittstelle. Danach erfolgte die nochmalige Überprüfung der korrekten Größe mittels Agarose-gelelektrophorese und Fragmente mit der gewünschten Insertlänge (200 bis 600 bp) wurden wie vorstehend beschrieben aus dem Gel eluiert.

### (K) Unidirektionale Klonierung

Die eluierten Fragmente wurden anschließend in ein BlueScript-Derivat (pBSC-NTER; Fa. Stratagene, Deutschland, Heidelberg), das so modifiziert wurde, daß es die beiden zu den Schnittstellend der cDNA-Fragmente kompatiblen Schnittstellen in seinem Polylinker enthält und mit den entsprechenden Restriktionsenzymen geschnitten wurde, gerichtet ligiert. Anschließend wurde in kompetente DH10B-Zellen (GibcoBRL LifeTechnologies GmbH, Karlsruhe) transformiert. Die cDNA-Insertionen wurden zur Kontrolle mit den PCR-Primern "NPCR1" (5'-TCG AGC GGC CGC CCG GGC AGG T-3') und "NPCR2" (5'-AGC GTG GTC GCG GCC GAG GT-3'), die auf den beiden Adaptern liegen, amplifiziert, ohne daß dabei zusätzliche Vektoranteile mitamplifiziert wurden.

## Patentansprüche

1. Verfahren zur Herstellung einer Genbibliothek, wobei das Verfahren die folgenden Schritte umfaßt:
(a) Herstellen einer doppelsträngigen cDNA aus einer mRNA-Population, wobei für die Synthese des ersten cDNA-Strangs ein Primer verwendet wird, der an seinem 5'-Ende einen ersten Bindungspartner eines Bindungspaars enthält, der eine Affinität zu einem zweiten Bindungspartner des Bindungspaars aufweist, an seinem 3'-Ende eine zu dem poly(A)-Schwanz der mRNA komplementäre poly(dT)-Sequenz und zwischen dem 5'-Ende, an das der erste Bindungspartner geknüpft ist und der poly(dT)-Sequenz eine Sequenz, die als Doppelstrang eine Erkennungsstelle für ein Restriktionsenzym des Typs II umfaßt;
(b) Fragmentieren der in Schritt (a) erhaltenen cDNA;
(c) Binden der erhaltenen Fragmente an den zweiten Bindungspartner, der an einen festen Träger gebunden ist;
(d) Ligieren eines doppelsträngigen Adaptermoleküls an das dem Ende mit dem ersten Bindungspartner gegenüberliegende Ende des Fragments, wobei das Adaptermolekül eine Schnittstelle für ein zweites Restriktionsenzym enthält;
(e) Durchführen einer in vitro-Amplifikation der an den Träger gebundenen Fragmente mit einem Primerpaar, wobei der erste Primer des Primerpaars im wesentlichen zu einem Strang des Adaptermoleküls komplementär ist und der zweite Primer des Primerpaars an seinem 5'-Ende einen ersten Bindungspartner umfaßt, der eine Affinität zu einem zweiten Partner eines Bindungspaars aufweist, und dessen Sequenz im wesentlichen der Sequenz des Primers aus Schritt (a) oder einer dazu komplementären Sequenz entspricht; und
(f) Ligieren der erhaltenen Produkte in einen Vektor.

2. Verfahren nach Anspruch 1, wobei nach Schritt (b) die Fragmente mit der gewünschten Länge isoliert werden.

3. Verfahren nach Anspruch 1 oder 2, wobei nach Schritt (b) die erhaltenen (isolierten) Fragmente mit glatten Enden versehen werden.

4. Verfahren nach einem der Ansprüche 1 bis 3, wobei die Amplifikationsprodukte aus Schritt (e) mit einem Restriktionsenzym des Typs II, das die in Schritt (a) eingeführte Restriktionsschnittstelle erkennt, gespalten werden.

5. Verfahren nach Anspruch 4, wobei danach ein Adaptermolekül an das 5'-Ende der PCR-Produkte, das nach Spaltung mit dem Restriktionsenzym des Typs II erhalten wurde, ligiert wird.

6. Verfahren nach Anspruch 5, wobei die Produkte mit einem zweiten Restriktionsenzym, das die in Schritt (d) eingeführte Schnittstelle erkennt, gespalten werden.

7. Verfahren nach Anspruch 1, wobei das Bindungspaar Biotin/Avidin oder Biotin/Streptavidin ist.

8. Verfahren nach Anspruch 1, wobei das Restriktionsenzym des Typs II aus der Gruppe BpmI, AlwI, BpsI, BpvI, Bci VI, BsaI, Bse RI, BsgI, Bsm Al, EarI, FokI, HgaI, HphI, MboII, PleI, SapI und SfaNI ausgewählt ist.

9. Verfahren nach einem der Ansprüche 1 bis 8, wobei der Primer für die Erststrangsynthese ein Gemisch von Primern ist, die 3' zu der poly(dT)-Sequenz die Sequenz 5'-VN-3' aufweisen, wobei V A,C oder G und N A, C, G oder T ist.

10. Verfahren nach einem der Ansprüche 1 bis 3, wobei die Fragmentierung in Schritt (b) eine Zufallsfragmentierung oder enzymatische Fragmentierung ist.

11. Verfahren nach Anspruch 10, wobei die Fragmentierung eine Ultraschallbehandlung oder enzymatischer (Teil-)Verdau ist.

12. Verfahren nach einem der Ansprüche 1 bis 11, wobei die Länge der Fragmente in Schritt (b) in einem Bereich von 200 bis 600 bp liegt.

13. Verfahren nach einem der Ansprüche 1 bis 12, wobei der feste Träger in Schritt (c) paramagnetische Perlen sind.

14. Verfahren nach einem der Ansprüche 1 bis 13, wobei das Restriktionsenzym in Schritt (d) ein Restriktionsenzym ist, das überstehende Enden erzeugt und/oder sich von dem Restriktionsenzym des Typs II aus Schritt (a) unterscheidet.

15. Verfahren nach einem der Ansprüche 1 bis 14, wobei die in vitro-Amplifikation in Schritt (e) eine PCR ist.

16. Verfahren nach Anspruch 4 und 5, wobei das Spalten so erfolgt, daß das erhaltene Fragment ein überstehendes Ende mit mindestens 2 Adeninresten aufweist und das Adaptermolekül gemäß Anspruch 5 ein dazu komplementäres überstehendes Ende aufweist.

17. Genbibliothek, die nach einem Verfahren nach einem der Ansprüche 1 bis 16 erhältlich ist.

18. Genbibliothek nach Anspruch 17, umfassend mindestens eine Sequenz eines Gens oder eines Teils davon anwesend ist, das für ein Protein kodiert, das bei einem der folgenden Prozesse eine Rolle spielt, ausgewählt aus der Gruppe: Aminosäuresynthese, zellulärer Metabolismus, Energiemetabolismus, Fettsäure- und Phospholipidmetabolismus, Purin-, Pyrimidin-, Nukleosid- und Nukleotidaufbau und -abbau, DNA-Replikation, Transkription, Translation, Proteintransport oder Proteinbindung,
**dadurch gekennzeichnet, daß** die Genbibliothek mindestens 50 Sequenzen umfaßt und davon mindestens 95% der Sequenzen der anwesenden Gene oder den Teilen davon zwischen 200 und 600 Basenpaaren lang sind.

19. Genbibliothek nach Anspruch 17 oder 18, wobei Sequenzen von mindestens 200 Genen oder Teilen davon anwesend sind, deren Produkte bei gleichen oder unterschiedlichen in Anspruch 1 definierten Prozessen eine Rolle spielen.

20. Genbibliothek nach einem der Ansprüche 17 bis 19, wobei Sequenzen von mindestens 500 Genen oder Teilen davon anwesend sind, deren Produkte bei gleichen oder unterschiedlichen in Anspruch 1 definierten Prozessen eine Rolle spielen.

21. Genbibliothek nach einem der Ansprüche 17 bis 20, wobei Sequenzen von mindestens 800 Genen oder Teilen davon anwesend sind, deren Produkte bei gleichen oder unterschiedlichen in Anspruch 1 definierten Prozessen eine Rolle spielen.

22. Genbibliothek nach einem der Ansprüche 17 bis 21, wobei die Gene oder Teile davon aus Maus, Ratte, Hund, Mensch, Schwein, Hamster oder Kuh stammen.

23. Genbibliothek nach einem der Ansprüche 17 bis 22, wobei mindestens 60% der Sequenzen Gene oder Teile davon umfassen, die aus dem 3'-Bereich der mRNA stammen.

24. Genbibliothek nach einem der Ansprüche 17 bis 23, wobei mindestens 60% der Gene oder der Teile davon Sequenzen umfassen, die keinen Poly(A)-Schwanz enthalten.

25. Genbibliothek nach einem der Ansprüche 17 bis 24, wobei die Sequenzen der Gene oder der Teile davon in einem prokaryontischen Plasmid vorhanden sind.

26. Genbibliothek nach einem der Ansprüche 17 bis 25, wobei die Sequenzen doppelsträngig sind.

27. Genbibliothek nach einem der Ansprüche 17 bis 26, wobei Sequenzen von mindestens 50 Genen oder Teilen davon vorhanden sind, die ausgewählt sind:
(a) aus den Sequenzen der SEQ.ID.-Liste "Replication",
(b) aus den Sequenzen der SEQ.ID.-Liste "Transcription",
(c) aus den Sequenzen der SEQ.ID.-Liste "Translation", und
(d) aus den Sequenzen der SEQ.ID.-Liste "Transport- und Bindeproteine".

28. Genbibliothek nach Anspruch 27, wobei Sequenzen von mindestens 200 Genen oder Teilen davon vorhanden sind, die ausgewählt sind :
(a) aus den Sequenzen der SEQ.ID.-Liste "Replication",
(b) aus den Sequenzen der SEQ.ID.-Liste "Transcription",
(c) aus den Sequenzen der SEQ.ID.-Liste "Translation", und
(d) aus den Sequenzen der SEQ.ID.-Liste "Transport- und Bindeproteine".

29. Genbibliothek nach Anspruch 28, wobei Sequenzen von mindestens 500 Genen oder Teilen davon vorhanden sind, die ausgewählt sind:
(a) aus den Sequenzen der SEQ.ID.-Liste "Replication",
(b) aus den Sequenzen der SEQ.ID.-Liste "Transcription", aus den Sequenzen der SEQ.ID.-Liste "Translation", und
(d) aus den Sequenzen der SEQ.ID.-Liste "Transport- und Bindeproteine".

30. Genbibliothek nach einem der Ansprüche 27 bis 29, wobei die Sequenzen von Genen aus mindestens zwei der Gruppen (a) bis (d) ausgewählt sind.

31. Genbibliothek nach einem der Ansprüche 17 bis 30, wobei Sequenzen von mindestens 50 Genen oder Teilen davon vorhanden sind, die ausgewählt sind aus den SEQ ID. NO. 1 bis SEQ ID. NO. 840.

32. Genbibliothek nach Anspruch 31, wobei Sequenzen von mindestens 200 Genen oder Teilen davon vorhanden sind, die ausgewählt sind aus den SEQ ID. NO. 1 bis SEQ ID. NO. 840.

33. Genbibliothek nach Anspruch 31 oder 32, wobei Sequenzen von mindestens 500 Genen oder Teilen davon vorhanden sind, die ausgewählt sind aus den SEQ ID. NO. 1 bis SEQ ID. NO. 840.

34. Transformante, die eine Genbibliothek nach einem der Ansprüche 17 bis 33 enthält.
